# EUROPEAN PATENT SPECIFICATION

(11) **EP 1 523 292 B1**
(45) Date of publication and mention of the grant of the patent: **16.05.2018**
(21) Application number: 03735337.2
(22) Date of filing: 03.07.2003
(51) Int. Cl.: A61F 5/44, A61F 5/451, A61L 17/00, F16L 9/00, A61M 39/00

(54) **CONDUIT DEVICE**
ROHRLEITUNG
TUBE

(30) Priority: 08.07.2002 DK 200201071
(43) Date of publication of application: 20.04.2005
(73) Proprietor: Coloplast A/S, 3050 Humlebaek (DK)
(72) Inventor: GULDFELDT, Signe, Uhre, 3400 Hillerod (DK); NIELSEN, Henrik, Lindenskov, 2765 Smørum (DK); JEPPESEN, Per, 2700 Brønshøj (DK)
(86) International application number: PCT/DK2003/000469
(87) International publication number: WO 2004/004611

(56) References cited:
- EP-A- 0 947 208
- US-A- 3 696 137
- US-A- 3 800 795
- US-A- 3 861 396
- US-A- 4 029 099
- US-A- 5 962 620
- US-A1- 2001 037 097
- US-A1- 2002 136 694

## Description

### Field of the invention

The present invention relates to a conduit device for conducting a fluid from a fluid source to a recipient, comprising a tubular portion having an inlet end and an outlet end, an inner side of said tubular portion being intended for contact with the fluid.

The invention furthermore relates to use of said conduit device as a hose member in connection with a collection bag, use of said conduit device as a hose member connecting a urinary catheter device with a urine collection bag, to a kit comprising such a conduit device and a collection bag, and to a urinary catheter arrangement comprising such a conduit device, a catheter device, and a collection bag.

### Background of the invention

Such conduit devices find widespread use within ia. the technical field of conducting and collecting body fluids. The conduit device may eg. form part of a kit or an arrangement, eg. a catheter arrangement for the relief of incontinence, and is e.g. utilized for connecting a urinary catheter device with a urine collection bag.

For instance, in the case of male urinary incontinence an external catheter device is generally placed externally on penis. The discharge spout of the catheter is connected with a hose member that in turn is connected with a urine collection bag. The wearer of the urinary catheter suitably places the urine collection bag in a position lower than the position of the urinary catheter to ensure that the urine effectively is drained off by gravity. To allow free movement of the wearer of the urinary catheter the urine collection bag is generally positioned at a part of the leg. Generally, it is preferred to position the urine collection bag at the shin to drain off urine in a standing as well as a sitting position. Furthermore, this position of the urine collection bag provides for the possibility of hiding it behind a trouser leg.

Basically, the same procedure is carried out in urinary catheterisation by insertion of one end of a catheter device through the urinary duct such that at least the tip thereof extends into the bladder. The other end of the catheter is connected with the inlet end of a hose member, the outlet end of which is connected with a collection bag as described in the above.

When using such catheter arrangements, the problem arises, following a discharge of urine, that the unbroken column of urine in the hose member gives rise to a considerable build-up of sub-pressure in the conduit device. This sub-pressure affects, in the case of an external urinary catheter device, the surface of the corona, and, in the case of a catheter device introduced into the bladder, the walls of the bladder, which may be sucked into abutment against the tip or inlet eyes of the catheter. In both of these cases, the wearer of the urinary catheter is subjected to discomfort, especially on extended exposure to the sub-pressure. In the case of external catheters, suction marks have been observed on the corona due the exposure to sub-pressure. In severe cases, oedema and subsequent infection can develop.

It has been suggested to overcome this problem by incorporating a vent or a valve in the urine collection bag or the lower part of the hose allowing the surrounding air to communicate with the interior of the catheter-hose-bag environment. Reference is made to e.g. US 3,835,857 and US 5,622,183. However, the position of a vent or valve in the bag or the lower part of the hose member entails the potential risk that urine, when the bag is full or about to be full, will escape the urine collection bag.

Although not concerned specifically with the same problem, WO 01/10362 discloses an arrangement for emptying a tubular conduit connecting a catheter and a urine measurement apparatus. This rather elaborate arrangement comprises a coupling element having a one-way valve and a double lumen hose, thereby preventing fluid to flow back to the bladder. By this arrangement, it is possible to empty the tubular conduit completely.

In connection with external catheter devices for the relief of male urinary incontinence, another suggestion is disclosed in US 5,897,540 wherein a slit-shaped opening is provided in a condom-like device for drainage of uncontrolled urine release. The opening is provided for ventilation of the space entrapped between the device and the penis and is placed in the position of use at the main body of the penis well behind the corona. The opening is not provided with a filter or similar means. A further suggestion is disclosed in US 4,656,675, which describes a condom-like device for draining off urine. The device is provided with a filter covering a hole positioned at a point close to the source of urine. The filter is capable of passing air but impervious to liquid at relative low pressures. A major drawback of this suggestion is that the filter is placed at an exposed position. The wearer of the device must be careful since the filter easily can be ripped off by abrasion or accident and the urine will tend to be conveyed through the hole in the device and not as intended through the hose member. Document US 3,861,396 discloses the features of the preamble of claim 1. In Applicant's international application WO03/056287 an external catheter device is disclosed, in which a membrane capable of selectively passing gases but retaining urine is fastened to the surface surrounding an aperture in the catheter. The membrane is at least partly protected by a shield.

As this arrangement entails that the amount of urine discharged from a wearer may be drained to a urine collection bag via a hose member without leaving a substantial amount of urine in the hose member giving rise to a sub-pressure at the surface of the corona, the arrangement thus provides a solution that functions satisfactorily when relieving male urinary incontinence by means of an external catheter device. However, this solution may not be applied in connection with other types of catheter devices. Furthermore, the arrangement is to some extent complicated to manufacture, and additionally, gases, ia. ambient air is introduced into the urine containing system, which entails a potential risk of infection. In addition, the collection bag is filled with gases when taking the arrangement into use.

### Summary of the invention

With this background it is an object of the present invention to provide a conduit device of the kind mentioned in the introduction, in which the formation of a sub-pressure in the tubular portion is eliminated or at least substantially reduced, and which at the same time is simple and inexpensive to manufacture.

It is a further object of the invention to provide a conduit device that may be utilized with a large variety of designs of the fluid source and the recipient, respectively.

These and further objects are met by the provision of a conduit device of the kind stated in the introduction and which is further defined in claim 1.

It turns out that the difference in surface tension between the inner side of the tubular portion and the fluid to be conducted therein is an important factor in the formation of a fluid column in the tubular portion and thus the resulting undesirable sub-pressure. In the case in which the conduit device is employed as a hose member forming the connection between a urinary catheter device and a urine collection bag, the fluid - urine - normally has a surface tension in the range of 55 to 65 dyn/cm (mN/m). In comparison, the polymer materials generally utilized for such hose members possess a substantially lower surface tension, typical examples being polyolefins, ethylene-vinyl acetate (EVA), PE, PE copolymer, PVC, PP etc. having a surface tension of typically less than 40 dyn/cm.

In conduit devices made from such traditional materials, the urine will form drops on the inner surface of the tubular portion. These drops form a plurality of more or less coherent columns that prevent emptying of the urine into the collection bag and thus causes sub-pressure in the inlet end of the tubular portion.

Without intending to limit the scope of the invention to a specific explanation, it is believed that in the conduit device according to the invention, in which means are provided for adapting the surface tension of at least the fluid, the tendency of the fluid to adhere to the inner side in the shape of drops is eliminated or at least substantially reduced. This prevents, in turn, that the fluid in the tubular portion forms the above-mentioned plurality of columns, without the need for introduction of gases from the surroundings. Consequently, the fluid is able to flow substantially easier in the conduit.

Said means may be provided as a surface active agent introduced into the fluid, said surface active agent being capable of reducing the surface tension of the fluid.

In an example, not forming part of the claimed invention, the surface active agent may be provided in the form of tablet in a dispensing unit positioned near or at the inlet end of the tubular portion.

The surface active agent is incorporated in the tubular portion of the conduit device, such that the surface active agent is in contact with at least the inner side of the tubular portion. The surface active agent may be an additive dissolved in the material that makes up the tubular portion, or it may be in the form of smaller or larger separate accumulations of molecules scattered throughout said material.

As a further alternative, the surface active agent may be provided by means of a gel or by injection of a liquid surface active agent. The surface active agent can e.g. be chosen from the group of polyphenylene oxide-polyethylene oxide copolymers, polyethylene glycol esters etc., but any suitable material having the desired properties may be used.

In order to reduce the formation of columns even further and thus to enhance the flow through the tubular portion, at least one part of the tubular portion has enlarged cross-sectional dimensions in relation to the remaining parts of the tubular portion, e.g. by making at least a part of the tubular portion corrugated.

In an example, not forming part of the claimed invention, the cross-section may include at least one incision. Hereby, it is believed that gases present in the tubular portion prevent fluid from entering and adhering to the inner side of the tubular portion in the area of the incision or incisions.

In an example, not forming part of the claimed invention, the incision or incisions may have a substantially V-shaped or U-shaped cross-section and/or extend in a spiral or curve.

Additionally, the conduit device may include a cord member extending substantially throughout the length of the conduit device.

In a simple manner, any remaining fluid column may be eliminated in an embodiment, in which the outlet end of the tubular portion has a widened cross-section in relation to the remaining parts of the tubular portion.

The widened cross-section is preferably provided by at least one obliquely extending cut at the outlet end.

In addition to the means for adapting the surface tension of the fluid itself, means may, in a further embodiment, be provided for adapting the surface tension of the inner side of at least the tubular portion of the conduit device.

Such means may be provided by application of a material having a surface tension that is substantially the same or larger than the surface tension of the fluid on at least the inner side of the tubular portion. With this embodiment, the tubular portion may be made mainly from traditional materials having the desired properties with respect to flexibility, durability and ease of manufacture.

The material may comprise a continuous layer, or at least one longitudinally extending portion, thus securing that at least a part of the inner side of the tubular portion that is in contact with the fluid has a larger surface tension than the fluid, thus preventing the formation of fluid columns.

Alternatively, at least one sector of the tubular portion may be comprised of said material.

Advantageously, the material is applied by means of co-extrusion with the tubular portion. This embodiment is particularly simple to manufacture, using only well known techniques. Such techniques comprise, i.a., plasma treatment and corona treatment. Other techniques may equally be used such as coating, UV activation, shrinking and others.

In a preferred embodiment, the material is provided as an additive to the material of the tubular portion, the additive being capable of migrating to at least the inner side of the tubular portion.

In a further development of the preferred embodiment, the tubular portion includes a protective layer or coating on the outer side. This feature ensures that the additive does not interfere with any further operations, such as welding, for e.g. connecting the tubular portion with other elements.

The material may comprise a hydrophilic polymer, such as polyethylene-vinyl alcohol copolymer, ethylene-vinyl acetate copolymer having a high content of vinyl acetate, polyether esters, acrylate copolymers, polyether amides, polyurethanes etc., but may comprise any suitable material having the desired properties.

Preferably, the material also comprises at least one polyphenylene oxide, polyethylene oxide copolymer, alkyl- and/or arylsulphonates, polyethylene glycol ester, fatty acid ester, fluorosurfactant and/or siliconesurfactant. More preferably, the material comprises at least one of PEG 400 distearate, PEG 400 dilaurate, Standapol (trademark of Cognis, Germany), preferably Standapol 1345 and/or Standapol 1480, Pluronic (trademark of BASF Corporation, Germany), preferably Pluronic L10, Atmer (Trademark of Uniqema, the Netherlands), preferably Atmer 100, and/or Zonyl (trademark of Dupont, USA), preferably Zonyl FSO-100 and/or Zonyl FSN-100.

Alternatively, the means may be provided by surface treatment of the inner side of the tubular portion, e.g. by corona treatment or similar surface treatment affecting the surface tension of the inner side of the tubular portion.

In another aspect of the intention, use of the conduit device as a hose member in connection with a collection bag is provided.

In a further aspect of the invention, a kit comprising a conduit device and a collection bag is provided.

In still another aspect of the invention, use of the conduit device as a hose member connecting a urinary catheter device with a urine collection bag, is provided.

In a still further aspect of the invention, a urinary catheter arrangement is provided, said urinary catheter arrangement comprising a catheter device, a conduit device, and a collection bag, the catheter device being an external urinary catheter for the relief of urinary incontinence, or a urinary catheter for insertion into the bladder.

### Brief description of the drawings

In the following the invention will be described in further detail with reference to the schematic drawings, in which
Fig. 1 shows a view of a conduit device according to an embodiment of the invention, further incorporating a connecting element;
Fig. 2 shows a perspective view, on a larger scale, of a part of the tubular portion of the conduit device in a first embodiment of the invention;
Fig. 3 shows a view corresponding to Fig. 2 of a second embodiment of the invention;
Fig. 4 shows a view corresponding to Fig. 2 of a third embodiment of the invention;
Fig. 5 shows a view corresponding to Fig. 2 of a fourth embodiment of the invention;
Fig. 6 shows an example, not forming part of the invention;
Fig. 7 shows a perspective view, on a larger scale, of the tubular portion of the conduit device in a sixth embodiment of the invention;
Fig. 8 shows a view corresponding to Fig. 2 of an example, not forming part of the invention;
Fig. 9 shows a view corresponding to Fig. 2 of an example, not forming part of the invention;
Fig. 10 shows a view corresponding to Fig. 2 of an example, not forming part of the invention;
Figs. 11a and 11b show, on a larger scale, details of embodiments of the conduit device according to the invention;
Fig. 12 shows a view of a urinary catheter arrangement in an embodiment of the invention; and
Fig. 13 shows a view corresponding to Fig. 11 of a urinary catheter arrangement in another embodiment of the invention.

### Detailed description of preferred embodiments

Throughout the various figures of the drawings, elements having the same or analogous function in the various embodiments of the conduit device of the invention have been indicated by identical reference numerals.

The conduit device according to the invention is suitable for conducting any fluid. More specifically it is suitable for conducting body fluid, in particular urine.

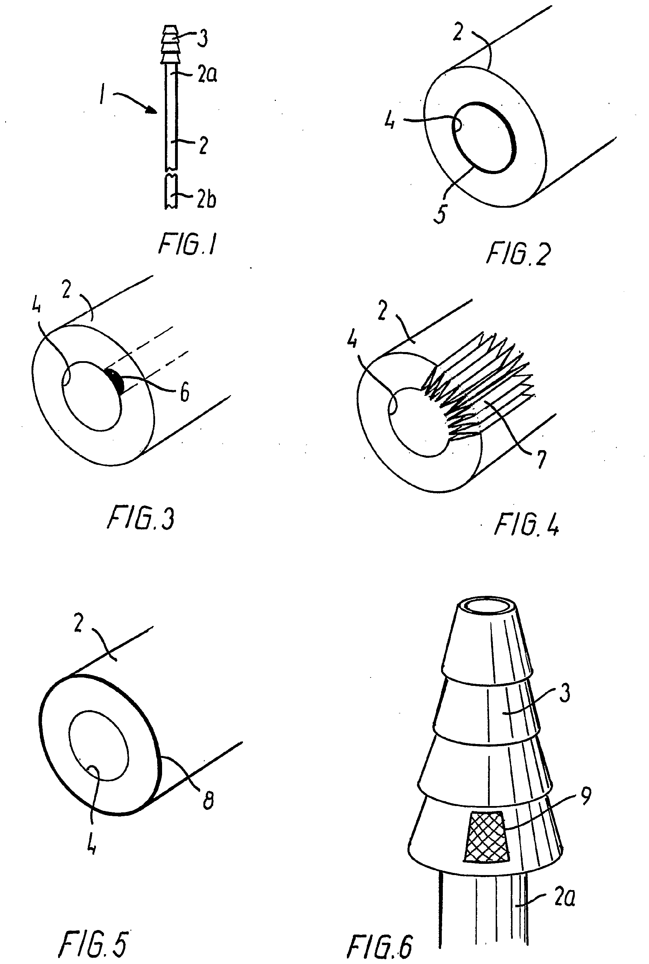

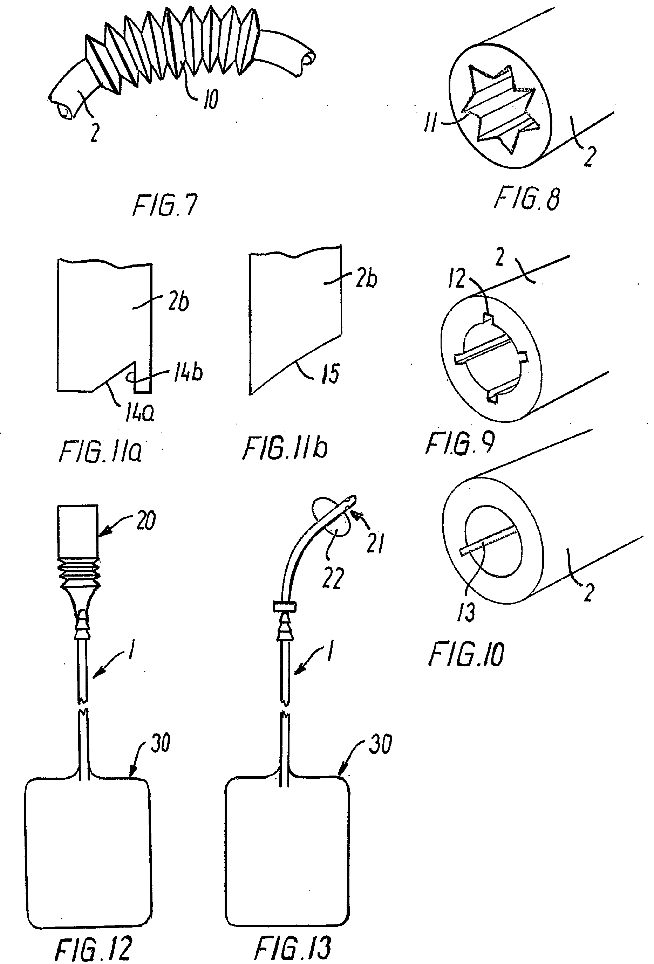

The conduit device shown in Figs. 1 to 5, 7 and 10 -13 of the drawings may advantageously be utilized as a hose member in a urinary catheter arrangement, e.g. as shown in Figs. 11 and 12. During bladder emptying discharges of urine may thus flow from the bladder constituting a fluid source via a catheter device to a recipient in the form of a urine collection bag through the conduit device, which connects the catheter device with the collection bag.

As shown in Fig. 1, the conduit device generally designated 1 has a longitudinally extending configuration and comprises a tubular portion 2 having an inlet end 2a that in a manner known per se is connected with a first connecting element 3, and an outlet end 2b. The connecting element 3 may be designed as a separate element or form an integral part of the tubular portion 2, and is intended for releasable coupling with the catheter device.

The tubular portion 2 is flexible but nevertheless resistant to forces resulting from eg. the user's movements which could lead to twisting and kinking of the tubular portion 2, thus blocking the flow of urine between the fluid source and the recipient. In the embodiment shown in Figs. 2 to 5, the tubular portion has a shape corresponding substantially to a hollow cylinder, the inner and outer surfaces being mainly even, whereas the cross-sectional dimensions of the tubular portion in the embodiments shown in Figs. 7 to 9 have inner and/or outer surfaces that are not mainly even. In this respect, it should furthermore be noted that the concept 'tubular' should be regarded as embracing any element of arbitrary outer dimensions having a through-going cavity of any cross-section.

The main constituent of the tubular portion 2 could be any suitable polymer that fulfils the demands with respect to flexibility, ease of manufacture etc., one example being eg. ethylene-vinyl acetate. As will be described in detail in the following, means are provided for adapting the surface tension of at least the fluid and possibly that of the inner side, ie. the side that in use is in contact with the fluid, of at least the tubular portion 2 such that the surface tension of at least the inner side is substantially the same or larger than the surface tension of the fluid. The means are not necessarily present in the tubular portion only. As will be described in further detail in connection with the embodiment of Fig. 6, the means may be provided in eg. an element connecting the conduit device with the fluid source. It is believed that this adaptation prevents the formation of fluid columns in the tubular portion due to the fact that the fluid cannot deposit on the inner side in the shape of droplets.

Various means for achieving the adaptation and/or for enhancing the effect are illustrated in Figs. 2 to 11 of the drawings.

The material may also be applied in one or more longitudinally extending portions on the inner side 4 of the tubular portion. In the embodiment shown in Fig. 3, one such portion 6 is illustrated. Such portions are preferably applied by means of co-extrusion with the remaining parts of the tubular portion 2. Each such portion 6 may, as indicated in Fig. 3, be flush with the inner side of the tubular portion, or protrude slightly into the channel delimited by the inner side 4 of the tubular portion 2. In the embodiment shown in Fig. 4, the material is applied in a sector 7 of the tubular portion 2 such that the material extends from the inner side to the outer side of the tubular portion 2. The material may be applied in any suitable pattern, eg. a spiral, a curve or a series of mutually overlapping portions around the inner surface of the tubular portion.

By forming at least a part of the tubular portion of a material having a different surface tension than the remaining parts, the formation of fluid columns is rendered almost impossible due to the difference in surface tension. However, out of considerations regarding manufacture and ia. strength and flexibility properties, this material should constitute less than approximately half the area of the entire tubular portions.

A slightly different manner of achieving the adaptation of the surface tension of the inner side of at least the tubular portion 2 is illustrated in the embodiment shown in Fig. 5. In this embodiment, the material is provided as an additive to the material of the tubular portion 2, the additive being capable of migrating to at least the inner side of the tubular portion 2. In order to prevent the additive from contacting the surroundings, the tubular portion 2 includes a protective layer or coating 8 on the outer side. Such a layer or coating may be applied in any suitable manner, and, when applied, ensures that the tubular portion may be connected with other elements without interfering with e.g. a welding process.

In a further embodiment, not illustrated, the means for altering the surface tension of the inner side of the tubular portion are provided by surface treatment of the inner side of the tubular portion, e.g. by means of corona treatment or similar surface treatment affecting the surface tension of the inner side of the tubular portion. A non-exhaustive list of examples of such treatments could be plasma treatment, chemical surface treatment etc.

Basically, the surface tension altering means are provided as a surface active agent added to the fluid, said surface active agent being capable of reducing the surface tension of the fluid. Such surface tension reducing agents are known per se and are preferably chosen from the group of polyphenylene oxide-polyethylene oxide copolymers, polyethylene glycol esters etc.

It is noted that the means for adapting the surface tension may be applied to the fluid only. However, it is preferred that such means, to be described in the following, are combined with means for adapting the surface tension of the inner side of at least a part of the tubular portion of the conduit device, as has been described in the above. It is particularly advantageous if the means, e.g. in the form of a material applied to the tubular portion, affects both the fluid and the inner side of the tubular member. For instance, material applied to the tubular material washes out into the fluid and thus affects the surface tension of both the inner side and the fluid, possibly in connection with a migration of the material within the tubular portion.

Fig. 6 illustrates an example making use of this principle. The surface active agent is provided in the form of tablet in a dispensing unit 9 formed in the first connecting element 3. The dispensing unit may be placed at other positions near or at the inlet end 2a of the tubular portion 2.

The surface active agent is incorporated in the tubular portion of the conduit device, such that the surface active agent is in contact with at least the inner side of the tubular portion. This may be carried out in the manner indicated in Figs. 2 to 4 and described in connection with the embodiment of Fig. 5 in respect of the coating or layer of a surface tension increasing material, or in any other manner. In case an inner coating or layer is provided, it can be this coating or layer that releases the surface active agent. Such a coating or layer may be applied in any suitable pattern, e.g. a spiral, a curve or a series of longitudinally extending portions or discrete spots around the inner surface of the tubular portion.

The surface active agent may be an additive dissolved in the material that makes up the tubular portion, or it may be in the form of smaller or larger separate accumulations of molecules scattered throughout said material.

As a further alternative, the surface active agent may be provided in the form of a gel or by injection into the lumen of the tubular portion of a liquid surface active agent.

In order to enhance the effect of the surface tension adaptation described in the above, the cross-section of the tubular portion may be designed in such a manner that the fluid is prevented from forming coherent fluid columns in the tubular portion. It is to be understood, however, that other parameters affect the flow through the conduit device as well. Hence, the position of use of the conduit device with regard to the fluid source and the recipient, respectively, has a noticeable influence on the flow, as gravity to some extent forces the fluid to flow downwards when the fluid source is situated at a high level than the recipient. Furthermore the flow is affected by the inner diameter of at least the tubular portion and by the roughness of the inner surface. In turn, the gravitational forces are affected by the inner diameter and the roughness of the inner surface. Consequently, in order to optimise the flow through the conduit device, all of the above parameters have to be considered.

In Fig. 7, this is achieved by forming at least a part of the tubular portion 2 with corrugations 10. A similar effect is obtained by examples shown in Figs. 8 and 9, in which the cross-section of the tubular portion 2 has an irregular inner side, preferably including a plurality of incisions 11 and 12, having a substantially V-shaped and U-shaped form, respectively, to form a substantially star-shaped cross-section. It is believed that the presence of air or gases in the incisions increases the security against the formation of fluid columns in the tubular portion, as any gas present in the incisions will hinder entrance of the fluid. The incisions may have any suitable shape such as the shown longitudinally extending shape or a spiral or curved shape, e.g. a single spiral incision extending substantially throughout the length of the tubular portion.

In the embodiment shown in Fig. 10, a cord member 13 extends within the tubular portion 2. The cord member, which may be solid or be designed as a hollow element, may be made from any suitable material and have a surface tension that is different, eg. higher than that of the tubular portion 2. The cord member 13 prevents or reduces the formation of fluid columns in the tubular portion by breaking the surface of the droplet which would otherwise form in the tubular portion. The cord member thus acts as a wick for the fluid.

Figs. 11a and 11b illustrate embodiments, by which the risk of fluid columns remaining in the conduit device is reduced even further by ensuring that the fluid accommodated in the tubular portion 2 flows off to the recipient. By forming the outlet end 2b of the tubular portion 2 with a widened cross-section in relation to the remaining parts of the tubular portion 2, any fluid column present in the tubular portion cannot exist in the outlet area due to the combined effect of the surface tension adaptation between the conduit device and the fluid passed therein and the widened outlet of the tubular portion. The widened cross-section of the outlet may in a simple manner be provided as shown in Fig. 11a, viz. by means of two obliquely extending cuts 14a, 14b at the outlet end, or as shown in Fig. 11b, by providing the entire outlet end 2b with an obliquely extending cut 15. The cut or cuts may have any shape, as long as the cross-sectional dimensions of the outlet end are increased.

The conduit device according to the invention may e.g. form part of a kit that further includes a collection bag. The kit may be used for conducting and receiving any fluid, but is particularly advantageous with body fluids, e.g. as wound drains or urinary catheterization.

As discussed in the above, the conduit device as described in connection with the embodiments of Figs. 1 to 5, 7 and 10-11 may advantageously be used as a hose member connecting a urinary catheter device with a urine collection bag.

In Fig. 12, a urinary catheter arrangement is shown, which comprises a catheter device 20, a conduit device 1 and a collection bag 30. The catheter device 20 is an external urinary catheter that is well known in the art for the relief of male urinary incontinence.

In Fig. 13, a urinary catheter arrangement is shown, which comprises a catheter device 21, a conduit device 1 and a collection bag 30. The catheter device 21 is a urinary catheter for insertion into the bladder and is, in a manner known per se, provided with a balloon member 22 for keeping the catheter device into place in the bladder.

### Example

The influence of a number of additives on the surface tension properties of a thermoplastic (EVA) was tested. The compositions were mixed in a Bra-bender mixer in 1/2 hour at 130°C and 60 rpm; after which the compositions were extruded into sheets between two PET-foils. With reference to table 1 below, the surface tension properties of each composition sheet were measured in terms of the contact angle between the tangent to the surface of a drop of water and the material surface in the point where these two surfaces meet. It was not possible to measure contact angles less than 30°; at this value, the water completely wets the material surface.

**Table 1**

| Additive | Contact angle |
|---|---|
| No additive | 107° |
| 3% PEG-400 distearate | 58° |
| 3% PEG-400 dilaurate | < 30° |
| 3% Pluronic L10 | 36° |
| 3% Standapol 1345 | < 30° |
| 10% Pebax MV1074 | 101° |
| 3% Atmer 100 | < 30° |

As may be seen, the test showed that PEG-400 dilaurate, Standapol and Atmer show excellent skill in raising the surface tension of the thermoplastic. However, PEG-400 distearate, Pluronic and, to a certain extent, Pebax also lowered the value of the contact angle.

This decrease of the contact angle may result from the surface tension properties of both the water and the thermoplastic composition or the water only. The surface tension properties of the water may change if the surface tension altering additives are being supplied to the water from the tubular portion. The invention should not be regarded as being limited to the embodiment described in the above but various modifications of and combinations between the various embodiments may be carried out without departing from the scope of the following claims.

## Claims

1. A urinary catheter arrangement comprising a catheter device, a collection bag, and a conduit device (1) for conducting a fluid from a fluid source via said catheter device to said collection bag (30), wherein said conduit device (1) comprises a tubular portion (2) having an inlet end (2a) and an outlet end (2b), an inner side of said tubular portion (2) being intended for contact with the fluid, **characterized in that** a surface active agent is provided for adapting the surface tension of at least the fluid, said surface active agent being provided such as to during use be able to be introduced into the fluid conducted in said tubular portion (2), said surface active agent being capable of reducing the surface tension of the fluid, wherein
said surface active agent is incorporated in the tubular portion (2) of the conduit device (1), such that the surface active agent is in contact with at least the inner side of the tubular portion (2), and wherein
at least one part of the tubular portion (2) has enlarged cross-sectional dimensions in relation to the remaining parts of the tubular portion (2), comprising at least one corrugated portion.

2. A urinary catheter arrangement according to claim 1, **characterized in that** the catheter device is an external urinary catheter for the relief of urinary incontinence.

3. A urinary catheter arrangement according to claim 1, **characterized in that** the catheter device is a urinary catheter for insertion into the bladder.

4. A urinary catheter arrangement as claimed in any of claims 1-3, in which the surface active agent is an additive dissolved in the material that makes up the tubular portion (2), or in the form of smaller or larger separate accumulations of molecules scattered throughout said material.

5. A urinary catheter arrangement as claimed in any of claims 1-3, in which said surface active agent comprises a gel or a liquid.

6. A urinary catheter arrangement as claimed in any one of claims 1 to 5, in which said surface active agent is chosen from the group of polyphenylene oxide-polyethylene oxide copolymers, polyethylene glycol esters etc.

7. A urinary catheter arrangement as claimed in any of the preceding claims, in which said conduit device (1) further includes acord member (13) extending substantially throughout the length of the conduit device (1).

8. A urinary catheter arrangement as claimed in anyone of the preceding claims, in which the outlet end (2b) of the tubular portion (2) has a widened cross-section in relation to the remaining parts of the tubular portion (2).

9. A urinary catheter arrangement as claimed in claim 8, in which the widened cross-section is provided by at least one obliquely extending cut (14a, 14b) at the outlet end.

10. A urinary catheter arrangement as claimed in anyone of the preceding claims, in which, additionally, means are provided for adapting the surface tension of the inner side of at least said tubular portion (2).

11. A urinary catheter arrangement as claimed in claim 10, in which said means are provided by application of a material having a surface tension that is substantially the same or larger than the surface tension of the fluid on at least the inner side of the tubular portion (2).

12. A urinary catheter arrangement as claimed in claim 11, in which said material (5) comprises a continuous layer.

13. A urinary catheter arrangement as claimed in claim 11, in which said material comprises at least one longitudinally extending portion (6).

14. A urinary catheter arrangement as claimed in claim 11, in which said material comprises at least one sector (7) of the tubular portion (2).

15. A urinary catheter arrangement as claimed in any one of claims 11 to 14, in which said material is applied by means of co-extrusion with the tubular portion (2).

16. A urinary catheter arrangement as claimed in claim 11, in which said material is provided as an additive to the material of the tubular portion (2), the additive being capable of migrating to at least the inner side of the tubular portion (2).

17. A urinary catheter arrangement as claimed in claim 16, in which the tubular portion (2) includes a protective layer or coating (8) on the outer side.

18. A urinary catheter arrangement as claimed in any of claims 11 to 17, in which said material comprises at least one polyphenylene oxide, polyethylene oxide copolymer, alkyl- and/or arylsulphona tes, polyethylene glycol ester, fatty acid ester, fluorosurfactant and/or siliconesurfactant.

19. A urinary catheter arrangement as claimed in claim 18, in which said material comprises at least one of PEG 400 distearate and/or PEG 400 dilaurate.

20. A urinary catheter arrangement as claimed in claim 1, in which said surface active agent is provided by surface treatment of the inner side of the tubular portion (2).

21. Use of the conduit device (1) of the urinary catheter arrangement of claim 1 for conducting a fluid from a fluid source to said collection bag (30).

## Patentansprüche

1. Harnkatheteranordnung, umfassend eine Kathetervorrichtung, einen Auffangbeutel und eine Leitungsvorrichtung (1), um ein Fluid von einer Fluidquelle über die Kathetervorrichtung zum Auffangbeutel (30) zu leiten, wobei die Leitungsvorrichtung (1) einen röhrenförmigen Abschnitt (2) mit einem Einlassende (2a) und einem Auslassende (2b) umfasst, wobei eine innere Seite des röhrenförmigen Abschnitts (2) für den Kontakt mit dem Fluid gedacht ist, **dadurch gekennzeichnet, dass** ein oberflächenaktiver Stoff vorgesehen ist, um die Oberflächenspannung mindestens des Fluids anzupassen, wobei der oberflächenaktive Stoff derart bereitgestellt ist, dass er während der Benutzung in das in dem röhrenförmigen Abschnitt (2) geleitete Fluid eingeführt werden kann, wobei der oberflächenaktive Stoff die Oberflächenspannung des Fluids zu reduzieren, wobei
der oberflächenaktive Stoff in dem röhrenförmigen Abschnitt (2) der Leitungsvorrichtung (1) eingegliedert ist, so dass der oberflächenaktive Stoff mit mindestens der inneren Seite des röhrenförmigen Abschnitts (2) in Kontakt ist, und wobei mindestens ein Teil des röhrenförmigen Abschnitts (2) vergrößerte Querschnittsabmessungen in Bezug auf die übrigen Teile des röhrenförmigen Abschnitts (2) hat und mindestens einen gewellten Abschnitt umfasst.

2. Harnkatheteranordnung nach Anspruch 1, **dadurch gekennzeichnet, dass** die Kathetervorrichtung ein äußerer Harnkatheter zur Linderung von Harninkontinenz ist.

3. Harnkatheteranordnung nach Anspruch 1, **dadurch gekennzeichnet, dass** die Kathetervorrichtung ein Harnkatheter zum Einführen in die Blase ist.

4. Harnkatheteranordnung nach einem der Ansprüche 1 - 3, wobei der oberflächenaktive Stoff ein in dem Material, aus dem der röhrenförmige Abschnitt (2) besteht, gelöstes Additiv oder in der Form kleinerer oder größerer Ansammlungen von im Material verstreuten Molekülen ist.

5. Harnkatheteranordnung nach einem der Ansprüche 1 - 3, wobei der oberflächenaktive Stoff ein Gel oder eine Flüssigkeit umfasst.

6. Harnkatheteranordnung nach einem der Ansprüche 1 bis 5, wobei der oberflächenaktive Stoff aus der Gruppe aus Polyphenylenoxid-Polyethylenoxid-Copolymeren, Polyethylenglykolestern usw. ausgewählt ist.

7. Harnkatheteranordnung nach einem der vorhergehenden Ansprüche, wobei die Leitungsvorrichtung (1) ferner ein Schnurglied (13) aufweist, das sich im Wesentlichen durch die gesamte Länge der Leitungsvorrichtung (1) erstreckt.

8. Harnkatheteranordnung nach einem der vorhergehenden Ansprüche, wobei das Auslassende (2b) des röhrenförmigen Abschnitts (2) einen in Bezug auf die übrigen Teile des röhrenförmigen Abschnitts (2) verbreiterten Querschnitt hat.

9. Harnkatheteranordnung nach Anspruch 8, wobei der verbreiterte Querschnitt durch mindestens einen sich schräg erstreckenden Schnitt (14a, 14b) am Auslassende bereitgestellt ist.

10. Harnkatheteranordnung nach einem der vorhergehenden Ansprüche, wobei zusätzlich Mittel vorgesehen sind, um die Oberflächenspannung der inneren Seite mindestens des röhrenförmigen Abschnitts (2) anzupassen.

11. Harnkatheteranordnung nach Anspruch 10, wobei die Mittel durch Aufbringen eines Materials mit einer Oberflächenspannung, die im Wesentlichen die gleiche wie des Fluids oder höher als die des Fluids ist, auf mindestens die innere Seite des röhrenförmigen Abschnitts (2) bereitgestellt sind.

12. Harnkatheteranordnung nach Anspruch 11, wobei das Material (5) eine durchgehende Schicht umfasst.

13. Harnkatheteranordnung nach Anspruch 11, wobei das Material mindestens einen sich in Längsrichtung erstreckenden Abschnitt (6) umfasst.

14. Harnkatheteranordnung nach Anspruch 11, wobei das Material mindestens einen Sektor (7) des röhrenförmigen Abschnitts (2) umfasst.

15. Harnkatheteranordnung nach einem der Ansprüche 11 bis 14, wobei das Material mittels Koextrusion mit dem röhrenförmigen Abschnitt (2) aufgebracht ist.

16. Harnkatheteranordnung nach Anspruch 11, wobei das Material als ein Additiv zu dem Material des röhrenförmigen Abschnitts (2) vorgesehen ist, wobei das Additiv in der Lage ist, zu mindestens der inneren Seite des röhrenförmigen Abschnitts (2) zu migrieren.

17. Harnkatheteranordnung nach Anspruch 16, wobei der röhrenförmige Abschnitt (2) eine Schutzschicht oder Beschichtung (8) an der Außenseite aufweist.

18. Harnkatheteranordnung nach einem der Ansprüche 11 bis 17, wobei das Material mindestens ein Polyphenylenoxid, Polyethylenoxid-Copolymer, Alkyl- und/oder Arylsulfonate, Polyethylenglykolester, Fettsäureester, Fluortensid und/oder Silicontensid umfasst.

19. Harnkatheteranordnung nach Anspruch 18, wobei das Material mindestens ein PEG-400 Distearat und/oder PEG-400 Dilaurat umfasst.

20. Harnkatheteranordnung nach Anspruch 1, wobei der oberflächenaktive Stoff durch Oberflächenbehandlung der inneren Seite des röhrenförmigen Abschnitts (2) bereitgestellt ist.

21. Verwendung der Leitungsvorrichtung (1) der Harnkatheteranordnung nach Anspruch 1 zum Leiten eines Fluids von einer Fluidquelle zu dem Auffangbeutel (30).

## Revendications

1. Agencement de cathéter urinaire comprenant un dispositif de cathéter, une poche de collecte, et un dispositif de tuyau (1) pour conduire un fluide depuis une source de fluide par le biais dudit dispositif de cathéter jusqu'à ladite poche de collecte (30), ledit dispositif de tuyau (1) comprenant une portion tubulaire (2) ayant une extrémité d'entrée (2a) et une extrémité de sortie (2b), un côté intérieur de ladite portion tubulaire (2) étant destiné à venir en contact avec le fluide, **caractérisé en ce qu'**un agent tensioactif est prévu pour adapter la tension de surface au moins du fluide, ledit agent tensioactif étant prévu de manière à pouvoir être introduit, au cours de l'utilisation, dans le fluide conduit dans ladite portion tubulaire (2), ledit agent tensioactif étant capable de réduire la tension de surface du fluide,
ledit agent tensioactif étant incorporé dans la portion tubulaire (2) du dispositif de tuyau (1) de telle sorte que l'agent tensioactif soit en contact avec au moins le côté intérieur de la portion tubulaire (2), et
au moins une partie de la portion tubulaire (2) ayant des dimensions en section transversale agrandies par rapport aux parties restantes de la portion tubulaire (2), comprenant au moins une portion ondulée.

2. Agencement de cathéter urinaire selon la revendication 1, **caractérisé en ce que** le dispositif de cathéter est un cathéter urinaire extérieur pour soulager l'incontinence urinaire.

3. Agencement de cathéter urinaire selon la revendication 1, **caractérisé en ce que** le dispositif de cathéter est un cathéter urinaire destiné à être inséré dans la vessie.

4. Agencement de cathéter urinaire selon l'une quelconque des revendications 1 à 3, dans lequel l'agent tensioactif est un additif dissous dans le matériau constituant la portion tubulaire (2), ou sous la forme d'accumulations de molécules séparées plus petites ou plus grandes éparpillées dans tout ledit matériau.

5. Agencement de cathéter urinaire selon l'une quelconque des revendications 1 à 3, dans lequel ledit agent tensioactif comprend un gel ou un liquide.

6. Agencement de cathéter urinaire selon l'une quelconque des revendications 1 à 5, dans lequel ledit agent tensioactif est choisi parmi le groupe des copolymères d'oxyde de polyphénylène-oxyde de polyéthylène, des esters de polyéthylène glycol, etc.

7. Agencement de cathéter urinaire selon l'une quelconque des revendications précédentes, dans lequel ledit dispositif de tuyau (1) comprend en outre un organe de cordon (13) s'étendant sensiblement sur toute la longueur du dispositif de tuyau (1).

8. Agencement de cathéter urinaire selon l'une quelconque des revendications précédentes, dans lequel l'extrémité de sortie (2b) de la portion tubulaire (2) présente une section transversale élargie par rapport aux parties restantes de la portion tubulaire (2).

9. Agencement de cathéter urinaire selon la revendication 8, dans lequel la section transversale élargie est réalisée par au moins une découpe s'étendant obliquement (14a, 14b) au niveau de l'extrémité de sortie.

10. Agencement de cathéter urinaire selon l'une quelconque des revendications précédentes, dans lequel, en outre, des moyens sont prévus pour adapter la tension de surface du côté intérieur d'au moins ladite portion tubulaire (2).

11. Agencement de cathéter urinaire selon la revendication 10, dans lequel lesdits moyens sont prévus par application d'un matériau ayant une tension de surface qui est sensiblement la même ou qui est supérieure à la tension de surface du fluide sur au moins le côté intérieur de la portion tubulaire (2).

12. Agencement de cathéter urinaire selon la revendication 11, dans lequel ledit matériau (5) comprend une couche continue.

13. Agencement de cathéter urinaire selon la revendication 11, dans lequel ledit matériau comprend au moins une portion s'étendant longitudinalement (6).

14. Agencement de cathéter urinaire selon la revendication 11, dans lequel ledit matériau comprend au moins un secteur (7) de la portion tubulaire (2).

15. Agencement de cathéter urinaire selon l'une quelconque des revendications 11 à 14, dans lequel ledit matériau est appliqué par coextrusion avec la portion tubulaire (2).

16. Agencement de cathéter urinaire selon la revendication 11, dans lequel ledit matériau est prévu sous forme d'additif ajouté au matériau de la portion tubulaire (2), l'additif étant capable de migrer vers au moins le côté intérieur de la portion tubulaire (2).

17. Agencement de cathéter urinaire selon la revendication 16, dans lequel la portion tubulaire (2) comporte une couche ou un revêtement de protection (8) sur le côté extérieur.

18. Agencement de cathéter urinaire selon l'une quelconque des revendications 11 à 17, dans lequel ledit matériau comprend au moins un copolymère d'oxyde de polyphénylène et d'oxyde de polyéthylène, des sulfonates d'alkyle et/ou d'aryle, un ester de polyéthylène glycol, un ester d'acide gras, un tensioactif fluoré et/ou un tensioactif de silicone.

19. Agencement de cathéter urinaire selon la revendication 18, dans lequel ledit matériau comprend au moins du distéarate de PEG 400 et/ou du dilaurate de PEG 400.

20. Agencement de cathéter urinaire selon la revendication 1, dans lequel ledit agent tensioactif est fourni par un traitement de surface sur le côté intérieur de la portion tubulaire (2).

21. Utilisation du dispositif de tuyau (1) de l'agencement de cathéter urinaire selon la revendication 1 pour conduire un fluide depuis une source de fluide jusqu'à ladite poche de collecte (30).
